# DEMANDE DE BREVET EUROPEEN

(11) **EP 0 645 601 A1**
(43) Date de publication de la demande: **29.03.1995**
(21) Numéro de dépôt: 94470028.5
(22) Date de dépôt: 02.09.1994
(51) Int. Cl.: G01B 11/02, C21C 5/48, B22D 1/00, F27D 21/00

(54) **Procédé et dispositif de mesure de l'évolution de la longueur d'usure d'une tuyère**

(30) Priorité: 29.09.1993 FR 9311765
(71) Demandeur: SOLLAC, F-92800 Puteaux (FR)
(72) Inventeur: Bernard, Franck, F-69340 Francheville (FR); Fressengeas, Nicolas, F-57050 Le Ban Saint Martin (FR); Heitz, Eric, F-57110 Illange (FR); Maufoy, Jean, F-57070 Metz (FR); Terrak, Salah, F-57290 Seremange (FR); Chapellier, Philippe, F-57100 Thionville (FR); Goetz, Michel, F-57070 Metz (FR); Loubet, Jacky, F-57158 Montigny-Lès-Metz (FR); Protin, André, F-57700 Hayange (FR); Vanzo, Jean-Marc, F-57070 Metz (FR)
(74) Mandataire: Ventavoli, Roger

(57) **Abrégé**

Selon l'invention, on équipe la tuyère avant son installation sur le récipient, dans lequel elle va permettre l'introduction d'un fluide au sein d'un bain métallique, d'une fibre optique dont une première extrémité affleure l'extrémité de la tuyère débouchant à l'intérieur dudit récipient, et dont l'autre extrémité est connectée à un réflectomètre optique, on émet dans ladite fibre une impulsion lumineuse, on détecte l'impulsion de retour, on mesure le temps écoulé entre l'émission de ladite impulsion et la détection de ladite impulsion de retour et on en déduit la longueur initiale de la fibre, en ce que, pendant l'utilisation de ladite tuyère, on mesure de la même manière la longueur de ladite fibre, et en ce qu'on en déduit l'évolution de la longueur d'usure de ladite tuyère.

L'invention permet de contrôler le moment où le remplacement de la tuyère devient nécessaire.

## Description

L'invention concerne le domaine des récipients métallurgiques destinés à l'élaboration d'un métal liquide à haut point de fusion, tel que l'acier. Elle concerne plus particulièrement les tuyères (ou plus généralement les injecteurs) qui traversent la paroi de ces récipients à un niveau situé sous la surface du métal liquide (généralement dans le fond du récipient) et qui servent à introduire un fluide (gaz ou liquide) à l'intérieur dudit métal liquide, avec lequel elles sont donc mises au contact par leur extrémité.

Les fonds revêtus de réfractaire de nombreux récipients métallurgiques, tels que des convertisseurs ou, parfois, les fours électriques d'aciérie, sont traversés par des tuyères dont une extrémité débouche dans la partie du récipient appelée à recevoir le métal liquide. Dans le cas d'un convertisseur ou d'un four électrique d'aciérie, le gaz injecté par ces tuyères peut être de l'argon, ou de l'azote, et a pour fonction essentielle d'assurer un brassage du métal liquide (fonte ou acier). La tuyère peut alors être constituée par un simple tube en acier. Ce gaz peut aussi être de l'oxygène (éventuellement mélangé à un autre gaz ou à de la chaux en poudre), dont la fonction est de se combiner au carbone contenu dans le métal liquide, et d'assurer ainsi la transformation de la fonte liquide en acier, ou un complément de décarburation de l'acier liquide. L'oxygène se combine également au silicium et au phosphore présents dans le métal, ce qui permet de les éliminer totalement ou partiellement. Toutes ces réactions sont fortement exothermiques, et il faut refroidir les tuyères pour éviter leur destruction rapide. A cet effet, elles sont formées par deux tubes métalliques concentriques : l'oxygène est insufflé par le tube central (généralement en cuivre), et un fluide réfrigérant (fuel, gaz naturel, propane par exemple) circule dans l'espace annulaire ménagé entre les tubes et passe lui-même dans le métal liquide.

L'extrémité des tuyères affleure le fond du récipient, et les tuyères s'usent en principe en même temps que le revêtement réfractaire. En fait, ce fond tend à s'user de manière accélérée au voisinage des tuyères, car c'est une zone où les turbulences du métal liquide sont particulièrement intenses, et où les températures sont très élevées (de l'ordre de 2000°C) lorsque les tuyères insufflent de l'oxygène.

Il est donc important de connaître l'évolution de la longueur des tuyères au cours de la vie du fond du récipient. D'une part, il faut pouvoir détecter si ces tuyères se sont anormalement raccourcies, au point que des infiltrations de métal liquide à leur niveau risqueraient de mettre en question le bon fonctionnement et la sécurité de l'installation. D'autre part, en marche normale, l'usure des tuyères est aussi un bon indicateur de l'usure générale du fond du récipient.

Actuellement, l'usure des tuyères est le plus souvent évaluée par une méthode contraignante et imprécise. Elle consiste à déconnecter les tuyères de leur alimentation en gaz, et d'y introduire depuis l'extérieur du récipient une pige (ou ringard), constitué d'une longue tige dont l'extrémité comporte un renflement muni d'une arête. On fait progresser la pige en appuyant le renflement contre la paroi interne de la tuyère, et on arrête cette progression lorsqu'on sent une résistance subitement accrue qui traduit le fait que l'arête est sortie de la tuyère et a pu s'ancrer sur le réfractaire. On évalue alors la longueur restante de la tuyère d'après la longueur sur laquelle la pige a été enfoncé. Cette méthode est imprécise, car le résultat dépend dans une large mesure de la géométrie souvent très irrégulière du fond au niveau de l'extrémité de la tuyère. D'autre part, le contrôle ne peut être effectué que lors d'un arrêt de l'exploitation du récipient métallurgique, et on ne peut donc détecter immédiatement une usure devenue soudain anormalement importante et pouvant être dangereuse à très court terme. De plus, la mesure elle-même est longue : plus d'une minute par tuyère, ce qui impose un arrêt prolongé de l'installation si celle-ci comporte un grand nombre de tuyères (16 est un nombre assez courant dans un convertisseur). Enfin, cette méthode nécessite qu'un opérateur s'approche très près du fond du récipient, ce qu'il est difficile de faire en toute sécurité.

Le but de l'invention est de proposer une méthode simple, fiable et rapide pour la mesure de la longueur d'usure d'une tuyère d'un récipient métallurgique.

L'invention a pour objet un procédé de mesure de l'évolution de la longueur d'usure d'une tuyère pour l'introduction d'un fluide dans un métal liquide contenu dans un récipient métallurgique, caractérisé en ce qu'on équipe ladite tuyère, avant son installation sur ledit récipient, d'une fibre optique dont une première extrémité affleure l'extrémité de la tuyère débouchant à l'intérieur dudit récipient, et dont l'autre extrémité est connectée à un réflectomètre optique, en ce que, à l'aide dudit réflectomètre, on émet dans ladite fibre une impulsion lumineuse, on détecte l'impulsion de retour résultant de la réflexion de ladite impulsion sur ladite première extrémité de la fibre, on mesure le temps écoulé entre l'émission de ladite impulsion et la détection de ladite impulsion de retour et on en déduit la longueur initiale de la fibre, en ce que, pendant l'utilisation de ladite tuyère, on mesure de la même manière la longueur de ladite fibre, et en ce que, par comparaison avec la longueur initiale, on en déduit l'évolution de la longueur d'usure de ladite tuyère.

L'invention a également pour objet un dispositif pour la mesure de l'évolution d'usure de la longueur d'une tuyère pour l'introduction d'un fluide dans un métal liquide contenu dans un récipient métallurgique, cette tuyère comportant au moins un tube métallique, caractérisé en ce qu'il comprend au moins une fibre optique fixée sur le tube, et dont une extrémité affleure l'extrémité de ce tube qui débouche à l'intérieur du récipient, et un réflectomètre optique auquel est connectée l'autre extrémité de la fibre optique.

Comme on l'aura compris, l'invention consiste à intégrer une fibre optique à la tuyère dont on veut contrôler la longueur, et à mesurer la longueur résiduelle de ladite fibre par une méthode basée sur la réflectométrie optique. L'usure de la fibre accompagnant celle de la tuyère, le résultat de cette mesure est représentatif de la longueur de la tuyère contrôlée et, par comparaison avec la valeur de la longueur de la tuyère à l'état neuf, on en déduit à tout moment la diminution de longueur, donc l'importance de la partie usée. Cette mesure peut être effectuée à tout moment, y compris pendant l'utilisation du récipient métallurgique.

L'invention sera mieux comprise à la lecture de la description qui suit, faisant référence aux figures annexées suivantes :
- la figure 1, qui schématise un fond de récipient métallurgique à l'état neuf équipé d'une tuyère et d'un dispositif selon l'invention ;
- la figure 2 qui schématise le même fond de récipient métallurgique, à l'état usagé.

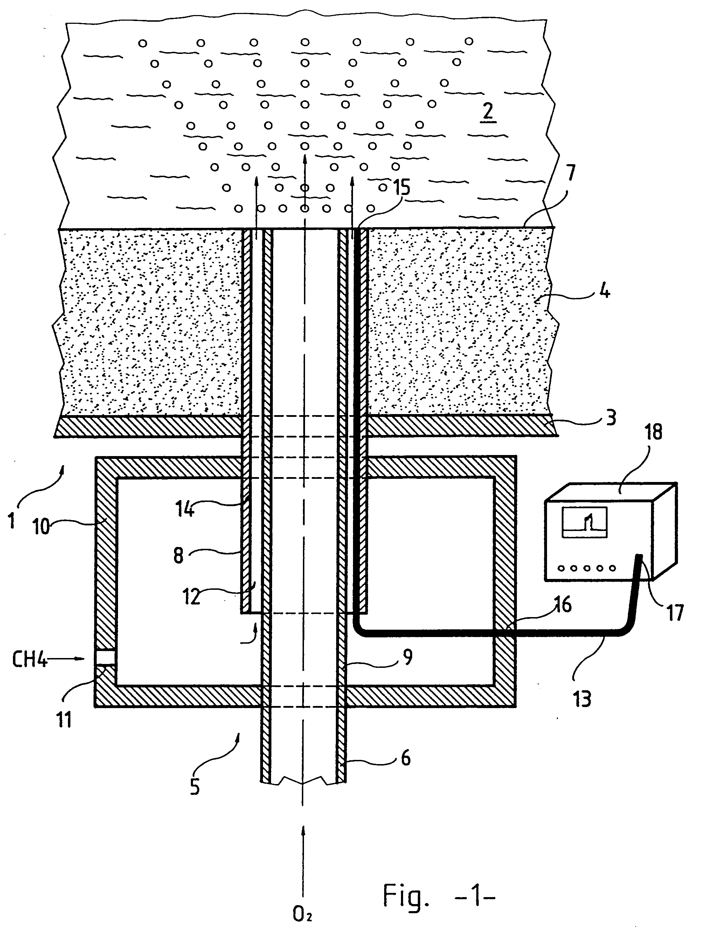

La figure 1 représente schématiquement le fond 1 d'un convertisseur (ou d'un four électrique) d'aciérie, renfermant un métal (acier ou fonte) liquide 2 que l'on désire décarburer. Ce fond 1 est constitué par une carcasse métallique 3 revêtue intérieurement d'une couche 4 de matériau réfractaire. Ce fond 1 est équipé d'une multiplicité de tuyères 5 adaptées à l'insufflation d'oxygène dans le métal liquide 2, dont l'une est représentée ici. D'une manière classique, cette tuyère 5 est formée par deux tubes concentriques :
- un tube intérieur 6 en un matériau métallique de préférence bon conducteur de la chaleur, tel que le cuivre, de diamètre intérieur 28 mm et de diamètre extérieur 39,4 mm (ces dimensions, comme les suivantes, n'étant données qu'à titre d'exemple), dont une extrémité affleure la surface externe 7 du réfractaire 4, et dont l'autre extrémité dépasse largement à l'extérieur de la carcasse métallique 3, et est reliée à une installation de distribution d'oxygène (non représentée) ;
- un tube extérieur 8 en un matériau métallique de préférence moins bon conducteur que le précédent, tel que l'acier, de diamètre intérieur 40 mm et de diamètre extérieur 47,5 mm, dont une extrémité affleure elle aussi la surface externe 7 du réfractaire 4, et dont l'autre extrémité dépasse à l'extérieur de la carcasse métallique 3, mais sur une longueur plus faible que le tube intérieur 6.

Le centrage des deux tubes 6 et 8 est assuré par des nervures non représentées, ménagées sur la paroi externe 9 du tube intérieur 6. Elles portent localement le diamètre extérieur du tube intérieur 6 à une valeur égale au diamètre intérieur du tube extérieur 8.

La tuyère comprend également, toujours de manière connue, une enceinte cylindrique 10 entourant la tuyère 5 sur une partie de sa portion dépassant à l'extérieur de la carcasse 3. L'extrémité dépassante du tube extérieur 8 débouche à l'intérieur de l'enceinte 10, alors que le tube intérieur 6 traverse l'enceinte 10 de part en part. Une étanchéité est assurée entre l'enceinte 10 et les tubes 6 et 8, et l'enceinte 10 est munie d'un orifice 11 à travers lequel on injecte un fluide refroidissant tel que du méthane (du fuel domestique, du propane, etc..., peuvent aussi être utilisés). Ce fluide refroidissant peut ainsi s'engouffrer dans l'espace 12 laissé libre entre les tubes 6 et 8 de la tuyère, et refroidir la tuyère 5. Sans cela, celle-ci serait portée à des températures excessives qui la détruiraient rapidement, suite à la combustion de l'oxygène qui a lieu lors de sa pénétration dans le métal liquide 2.

Selon l'invention, une fibre optique 13 est intégrée à la tuyère 5 lors de l'assemblage de celle-ci. Elle est fixée, par exemple comme on le voit sur les figures, à la paroi interne 14 du tube extérieur 8. Cette fixation peut être réalisée par collage, ou alors en ménageant dans la paroi 14 une rainure dont la largeur est légèrement inférieure au diamètre extérieur nominal de la fibre 13, et dans laquelle on fait pénétrer celle-ci de force, de manière à l'y coincer. Lors de sa mise en place, la longueur de la fibre 13 est ajustée de telle manière que l'une de ses extrémités 15 est exactement au même niveau que l'extrémité de la tuyère 5 qui affleure la surface externe 7 du revêtement réfractaire 4. D'autre part, la fibre 13 traverse une paroi de l'enceinte 10 en passant à travers un orifice 16, des moyens adéquats étant prévus pour assurer une étanchéité entre la fibre 13 et l'orifice 16. L'autre extrémité 17 de la fibre 13 est connectée à un réflectomètre optique 18.

L'invention est mise en oeuvre de la manière suivante. Conformément au principe connu de la réflectométrie optique, le réflectomètre 18 envoie dans la fibre 13 une courte impulsion lumineuse. Cette impulsion se propage dans la fibre 13, et à l'extrémité 15 de la fibre 13, elle rencontre l'interface fibre-métal (ou fibre-air si l'essai a lieu lorsque le récipient métallurgique 1 est vide). Une grande fraction de l'énergie lumineuse passe alors dans le métal 2 (ou dans l'air). Mais le restant est réfléchi dans la fibre 13 et guidé vers le réflectomètre 18, qui comporte des moyens pour détecter cette impulsion de retour et des moyens pour calculer le temps écoulé entre l'émission de l'impulsion et la détection de l'impulsion de retour. Connaissant la vitesse de propagation de l'impulsion dans la fibre 13, on peut en déduire la longueur de la fibre 13, avec une précision qui dépend de la résolution du réflectomètre 18. Pour les meilleurs d'entre eux, qui émettent des impulsions lumineuses de l'ordre du centimètre et dont l'horloge a une précision de 1 picoseconde, la longueur de la fibre 13 peut être donnée au mm près. On effectue d'abord une mesure de la longueur initiale de la fibre 13 juste après la mise en place de la tuyère 5. Puis on effectue périodiquement des mesures de cette longueur pendant l'utilisation du récipient 1, en admettant que la fibre 13 s'use à la même vitesse que la tuyère 5, et que la différence entre la longueur mesurée pour la fibre 13 et sa longueur initiale représente l'usure de la tuyère 5 depuis sa mise en place. Cette condition est très bien réalisée en particulier lorsque la température de fusion du matériau constituant la fibre 13 est égale ou de peu inférieure à celle du bain de métal liquide 2. Dans le cas de l'acier liquide, cette température est de l'ordre de 1600-1700°C. Or les fibres optiques en silice ont un point de fusion de 1500°C environ, ce qui les rend particulièrement aptes à cet usage. D'autre part elles ont un coefficient d'expansion thermique très proche de ceux de l'acier et du cuivre constituant la tuyère. Dans ces conditions, on est assuré que les variations de longueur de la fibre 13 mesurées par le réflectomètre 18 sont bien représentatives des variations de longueur de la tuyère 5 elle-même. L'appareillage est capable de détecter aussi bien les raccourcissements de la tuyère dus à son usure que ses allongements dus à sa dilatation lors de la mise en régime thermique du revêtement réfractaire 4.

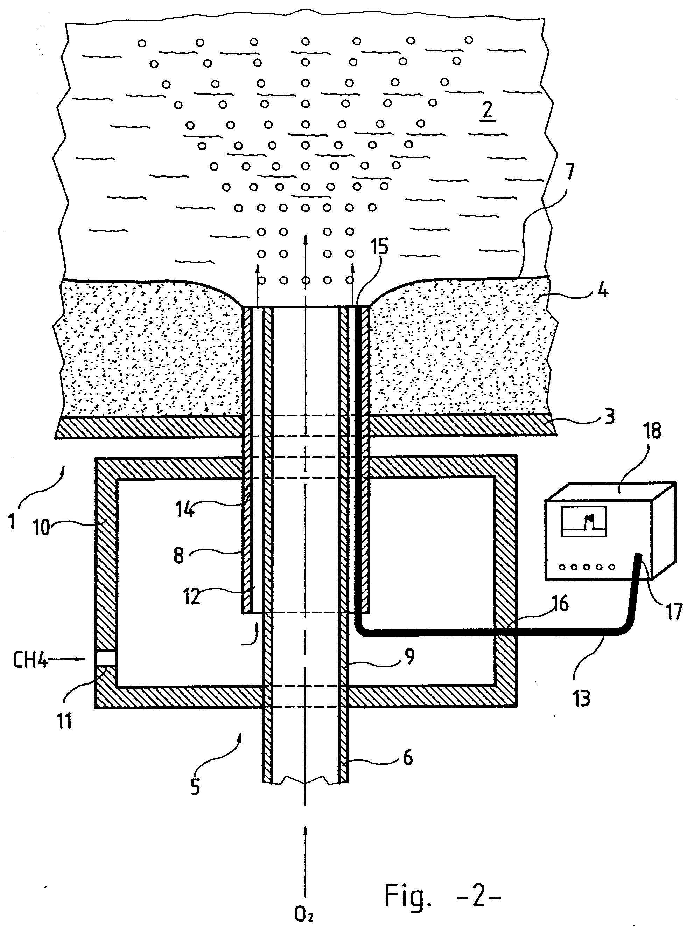

La figure 2 montre le fond 1 du convertisseur (ou du four électrique) après une utilisation prolongée ayant entraîné une usure du réfractaire 4 et de la tuyère 5. Pour les raisons évoquées plus haut (turbulences et température du métal accrues à ce niveau), l'usure du revêtement 4 est particulièrement prononcée au voisinage immédiat de la tuyère 5 d'où l'intérêt de disposer d'un moyen de mesure spécifique de la longueur de la tuyère.

On n'a décrit et représenté jusqu'ici qu'une tuyère équipée d'une fibre unique, et connectée seule à un réflectomètre. Mais, bien entendu, il est souhaitable que toutes les tuyères implantées dans le récipient métallurgique soient ainsi équipées et reliées chacune à un réflectomètre différent. Elles peuvent aussi être toutes reliées à un réflectomètre unique mais capable d'alimenter simultanément ou successivement une pluralité de fibres optiques en énergie lumineuse, d'analyser les impulsions de retour qu'elles lui transmettent et de restituer à l'opérateur les résultats de ces analyses. De même, il peut être recommandé d'implanter au moins deux fibres indépendantes dans chaque tuyère, de manière à pouvoir continuer les mesures même en cas de rupture accidentelle d'une fibre en cours d'utilisation.

Il est recommandé d'utiliser des fibres optiques en silice monomodes, dont le faible diamètre de coeur (9 µm) permet de minimiser l'intensité lumineuse provenant du métal liquide qui est collectée par la fibre et envoyée, elle aussi, au réflectomètre. Une intensité lumineuse trop forte pourrait, en effet, aveugler le réflectomètre. Si on désire (comme on l'a décrit et représenté) implanter ces fibres dans une tuyère pour insufflation d'oxygène, en les insérant entre les deux tubes constituant la tuyère, il faut prévoir des fibres suffisamment minces (par exemple de diamètre 0,9 mm, gaine plastique comprise). Pour fiabiliser l'installation, on peut éventuellement, peu après leur sortie des tuyères, prolonger ces fibres minces par des fibres plus épaisses, donc plus résistantes, que l'on relie au réflectomètre. Dans le cas où on veut instrumenter une tuyère monotube pour insufflation d'un gaz neutre, la fibre peut être fixée sur la paroi intérieure du tube et avoir un diamètre plus élevé que dans le cas précédent, puisqu'il y a davantage de place disponible pour l'installer sans perturber l'insufflation du gaz.

Les mesures de longueur des différentes tuyères ainsi instrumentées peuvent être effectuées avec une périodicité laissée à l'appréciation de l'opérateur, y compris pendant le fonctionnement même du récipient métallurgique. On peut ainsi détecter des usures brutales et imprévisibles des tuyères (de l'ordre du cm) qui accompagnent une usure similaire du réfractaire dans lequel elles sont implantées. On supprime ainsi l'opération de contrôle manuel de l'usure des tuyères qui est habituellement effectuée lors des arrêts de l'exploitation du récipient métallurgique, et dont on a exposé les inconvénients : perte de temps, donc de productivité de l'installation, et manque de précision de la mesure.

On notera que la fibre optique 13 peut être placée aussi bien sur la paroi intérieure du tube 8, comme le montrent les figures, que sur sa paroi extérieure.

Bien entendu, l'invention ne s'adresse pas seulement aux récipients destinés au traitement de la fonte ou de l'acier liquide (convertisseurs à oxygène, convertisseurs de type AOD pour l'élaboration des aciers inoxydables, fours électriques à arc). Elle s'adresse à tout récipient métallurgique dans lequel on utilise, pour l'introduction d'un fluide quelconque dans le métal liquide, des tuyères susceptibles de s'user. Il suffit de choisir le matériau constituant la fibre en fonction de sa température de fusion. Celle-ci doit être égale ou de peu inférieure à la température du métal traité, afin que l'usure de la fibre soit représentative de l'usure de la tuyère.

## Revendications

**1)** Procédé de mesure de l'évolution de la longueur d'usure d'une tuyère pour l'introduction d'un fluide dans un métal liquide contenu dans un récipient métallurgique, caractérisé en ce qu'on équipe ladite tuyère, avant son installation sur ledit récipient, d'une fibre optique dont une première extrémité affleure l'extrémité de la tuyère débouchant à l'intérieur dudit récipient, et dont l'autre extrémité est connectée à un réflectomètre optique, en ce que, à l'aide dudit réflectomètre, on émet dans ladite fibre une impulsion lumineuse, on détecte l'impulsion de retour résultant de la réflexion de ladite impulsion sur ladite première extrémité de la fibre, on mesure le temps écoulé entre l'émission de ladite impulsion et la détection de ladite impulsion de retour et on en déduit la longueur initiale de la fibre, en ce que, pendant l'utilisation de ladite tuyère, on mesure de la même manière la longueur de ladite fibre, et en ce que, par comparaison avec la longueur initiale, on en déduit l'évolution de la longueur d'usure de ladite tuyère.

**2)** Dispositif pour la mesure de l'évolution de la longueur d'usure d'une tuyère (5) pour l'introduction d'un fluide dans un métal liquide (2) contenu dans un récipient métallurgique, ladite tuyère comportant au moins un tube métallique (6,8), caractérisé en ce qu'il comprend au moins une fibre optique (13) fixée sur ledit tube (6,8), dont une extrémité (15) affleure l'extrémité dudit tube (6,8) débouchant à l'intérieur dudit récipient, et un réflectomètre optique auquel est connectée l'autre extrémité (17) de ladite fibre optique (13).

**3)** Dispositif selon la revendication 2, caractérisé en ce que ladite fibre optique (13) est fixée à ladite tuyère (5) par collage.

**4)** Dispositif selon la revendication 2, caractérisé en ce que une rainure est ménagée dans une paroi dudit tube (6,8), et en ce que ladite fibre optique (13) a été insérée de force dans ladite rainure.

**5)** Dispositif selon l'une des revendications 2 à 4, caractérisée en ce que ladite fibre optique (13) est en silice.

**6)** Dispositif selon l'une des revendications 2 à 5, caractérisé en ce que ladite fibre optique (13) est une fibre monomode.
